# EUROPEAN PATENT APPLICATION

(11) **EP 1 255 110 A2**
(43) Date of publication of application: **06.11.2002**
(21) Application number: 02253020.8
(22) Date of filing: 29.04.2002
(51) Int. Cl.: G01N 33/50, G01N 33/566, A61P 19/10

(54) **Methods and products for identifying modulators of P2X7 receptor activity, and their use in the treatment of skeletal disorders**

(30) Priority: 30.04.2001 US 287523; 04.04.2002 US 370054
(71) Applicant: Pfizer Products Inc., Groton, Connecticut 06340 (US)
(72) Inventor: Gabel, Christopher Allen, Groton, Connecticut 06340 (US); Ke, Hua Zhu, Groton, Connecticut 06340 (US)
(74) Representative: Hayles, James Richard

(57) **Abstract**

Disclosed are methods for screening compounds to identify therapeutic candidates for modulating bone mass and/or bone mineral density, as well as methods for treating skeletal disorders characterized by decreased bone mass and/or bone mineral density. The methods are based upon the identification and use of agonists of the P2X7 receptor.

## Description

### FIELD OF THE INVENTION

The present invention relates to the fields of molecular biology and osteology. More specifically, the invention relates to methods and products for screening compounds to identify therapeutic candidates for modulating bone mass and/or bone mineral density by identifying modulators of P2X7 receptor activity. Such therapeutic candidates may be useful in the treatment of skeletal disorders characterized by decreased bone mass and/or bone mineral density.

### BACKGROUND OF THE INVENTION

Cell surface ATP receptors can be divided into the metabotropic receptor family (P2Y/P2U receptor family) and the ionotropic receptor family (the P2X receptor family). Metabotropic receptor family members are G-protein coupled receptors and ionotropic receptor family members are ligand-gated channels. There are eleven metabotropic receptor family members and seven ionotropic receptor family members, P2X1 to P2X7.

The P2X7 receptor, like other members of the P2X receptor family, is an ATP-gated ion channel (Surprenant et al. (1996), *Science* 272:735-738; Rassendren et al. (1997), *J. Biol. Chem.* 272:5482-5486; Michel et al. (1998), *Br. J. Pharmacol.* 125:1194-1201). The P2X7 receptor, however, demonstrates attributes that clearly distinguish it from other members of the family. For example, the P2X7 receptor requires levels of ATP in excess of 1 mM to achieve activation whereas other P2X receptors activate at ATP concentrations less than or equal to 100 µM (Greenberg et al. (1988), *J. Biol. Chem.* 263:10337-10343; Steinberg et al. (1987), *J. Biol. Chem.* 262:8884-8888). The higher concentration requirement reflects, in part, the preference of the P2X7 receptor for ATP⁴⁻ as its ligand and the relatively low abundance of this species in media containing physiological concentrations of divalent cations (*e.g.*, Ca²⁺ and Mg²⁺). An additional unique feature of the P2X7 receptor is found in its conductance properties. All P2X receptors demonstrate non-selective channel-like properties following ligation, but the channels formed by the P2X7 receptor rapidly transform to pores that allow passage of solutes as large as 900 daltons (Steinberg et al. (1987), *J. Biol. Chem.* 262:8884-8888; Virginio et al. (1999), *J. Physiol.* 519:335-346). Molecular details of this transformation remain to be described, but domain swapping and deletion experiments have suggested that the carboxy terminal domain of the P2X7 receptor participates in pore complex formation; the carboxy terminal domain is significantly longer than the comparable domains in the other P2X receptors (North (1996), *Current Opin. Cell Biol.* 8:474-483). Possibly as a consequence of this pore-like activity, continuous ligation of the P2X7 receptor for times greater than fifteen minutes can lead to cell death (Di Virgilio (1995), *Immunol. Today* 16:524-528; Murgia et al. (1992), *Biochem. J*. 288:897-901; Ferrari et al. (1999), *FEBS Lett.* 447:71-75).

The P2X7 receptor displays a restricted cellular distribution, being observed primarily in cells of hematopoietic origin including monocytes and macrophages and some lymphocyte populations (Di Virgilio (1995), *Immunol. Today* 16:524-528; Collo et al. (1997), *Neuropharmacol*. 36:1277-1283). The receptor also has been reported to exist on microglial cells (Sanz et al. (2000), *J*. *Immunol.* 164:4893-4898), some cancer cells (Wiley et al. (1989), *Blood* 73:1316-1323), sperm (Foresta et al. (1996), *Am J. Physiol.* 270:C1709-C1714), and dendritic cells (Mutini et al. (1999), *J. Immunol*. 163:1958-1965). The P2X7 receptor has been reported to participate in a diverse list of cellular activities including lymphocyte proliferation (Baricordi et al. (1999), *J. Biol. Chem.* 274:33206-33208), fertilization (Foresta et al. (1996), *Am. J. Physiol.* 270:C1709-C1714), giant cell formation (Chiozzi et al. (1997), *J. Cell Biol.* 138:697-706), cell death (Murgia et al. (1992), *Biochem. J*. 288:897-901; Ferrari et al. (1999), *FEBS Lett*. 447:71-75), killing of invading mycobacteria (Lammas et al. (1997), *Immunity* 7:433-444), and IL-1 post-translational processing (Hogquist et al. (1991), *Proc. Natl. Acad. Sci. (USA)* 88:8485-8489; Perregaux et al. (1994) *J. Biol. Chem.* 269:15195-15203). Further, ligation of the P2X7 receptor has been associated with activation of phospholipase D and activation of some forms of NF-κB (Humphreys et al. (1996), *J. Immunol.* 157:5627-5637; Ferrari et al. (1997), *J. Cell Biol.* 139:1635-1643). It has been reported that the P2X7 receptor is expressed in rat bone tissue and in rat osteoclasts (Hoebertz et al. (2000) *Bone* 27: 503-510).

To address the role of the P2X7 receptor in IL-1β post-translational processing in whole animals, a P2X7 receptor-deficient mouse line has been generated (see Solle et al. (2001), *J. Biol. Chem.* 276:125-132). The P2X7 receptor-deficient mice exhibited normal production of pro-IL-1β in response to bacterial lipopolysaccharide stimulation of macrophages, and normal increases in peritoneal IL-6 in response to *in vivo* lipopolysaccharide injection. The P2X7 receptor deficient mice, however, did not exhibit normal externalization of mature IL-1β by ATP-stimulated macrophages, or normal increases in peritoneal IL-1 in response to ATP injection. Despite these differences, the P2X7 receptor-deficient mice are healthy and fertile and demonstrate no overt phenotype.

### SUMMARY OF THE INVENTION

The present invention provides methods for screening compounds to identify therapeutic candidates for modulating bone mass and/or bone mineral density. The invention also provides methods for treating skeletal disorders characterized by decreased bone mass and/or bone mineral density, based upon the identification and use of agonists of the P2X7 receptor. The methods for screening compounds depend, in part, upon the characterization of the role of the P2X7 receptor and its activities *in vivo*.

Thus, in one aspect, the present invention provides methods for screening compounds to identify therapeutic candidates for modulating bone mass and/or bone mineral density, by determining the ability of a compound to increase or decrease an indicator of P2X7 receptor activity. In these methods, a compound is contacted with the P2X7 receptor on the surface of cells from a cell line expressing the P2X7 receptor on their cell surfaces. The contacting step is conducted under conditions which, but for the presence of the compound, would permit the binding of the P2X7 receptor to a P2X7 receptor ligand. The indicator of P2X7 receptor activity is then measured; an increase or decrease in the indicator indicates that the compound is a candidate for modulating bone mass and/or bone mineral density by interacting with a P2X7 receptor *in vivo*.

In some embodiments, the compound is contacted with the P2X7 receptor without the addition of a known P2X7 receptor ligand to the assay to identify P2X7 receptor agonists. In these embodiments, an increase in the indicator of P2X7 receptor activity indicates that the compound is a candidate for an agonist of the P2X7 receptor. In other embodiments, the compound is added in addition to a known P2X7 receptor ligand to identify P2X7 receptor agonists and antagonists. In these embodiments, an increase in the indicator of P2X7 receptor activity indicates that the compound is a candidate for an agonist of the P2X7 receptor or an enhancer of the agonist activity of the known ligand, and a decrease in the indicator indicates that the compound is a candidate for an antagonist of the P2X7 receptor or an inhibitor of the agonist activity of the known ligand.

In any of the foregoing embodiments, the interaction between the compound or ligand and the receptor is determined by measuring an indicator of P2X7 receptor activity. Preferred indicators are increased cell permeability or increased intracellular accumulation of a macromolecule; increased IL-1α, IL-1β, or IL-18 post-translational processing in activated inflammatory cells; increased IL-6 production in inflammation-induced cells; L-selectin shedding; stress kinase activation; and lymphoproliferation. In some embodiments in which the cells expressing the P2X7 receptor binding domain are osteoclast progenitors, the indicator is differentiation into an osteoclast. In some embodiments in which the cells are osteoclasts, the indicator is increased resorption pit formation or increased apoptosis.

In any of the above-described embodiments, the cells expressing the P2X7 receptor can be cells which naturally express the P2X7 receptor or can be derived from a cell line that has been transformed with a genetic construct encoding the P2X7 receptor.

In another aspect, the present invention provides methods for screening compounds to identify therapeutic candidates for modulating bone mass and/or bone mineral density, by determining the ability of a compound to bind to a P2X7 receptor binding domain. In these methods, a compound is contacted with at least the binding domain of the P2X7 receptor on the surface of cells from a cell line expressing the binding domain of the P2X7 receptor on their cell surfaces. The contacting step is conducted under conditions which, but for the presence of the compound, would permit the binding of the P2X7 receptor binding domain to a P2X7 receptor ligand. Binding, if any, between the P2X7 receptor binding domain and the compound is detected. Binding between the P2X7 receptor binding domain and the compound indicates that the compound is a candidate for modulating bone mass and/or bone mineral density by interacting with a P2X7 receptor *in vivo*.

In some embodiments, the compound is contacted with the P2X7 receptor binding domain without the addition of a known P2X7 receptor ligand to the assay. In these embodiments, binding to the P2X7 receptor binding domain indicates that the compound is a candidate for interacting with the P2X7 receptor. In other embodiments, the compound is added in the presence of a known P2X7 receptor ligand. In these embodiments, either binding of the compound to the P2X7 receptor binding domain or a decrease in the binding of the known ligand to the P2X7 receptor binding domain indicates that the compound is a candidate for interacting with the P2X7 receptor.

In some embodiments of the preceding methods, in order to facilitate detection of binding amongst the compound, the known ligand, and/or the P2X7 receptor binding domain, either the compound or the known P2X7 receptor ligand are labeled with a detectable label. After contacting the labeled element with the P2X7 receptor binding domain on the cell surface, any unbound labeled element is washed away, and the labeled element bound to the P2X7 receptor binding domain is detected. Preferred labels in such assays are radioisotopes, fluorescent labels, chemiluminescent labels, enzymatic domains, specific binding elements, and metal atoms.

In any of the above-described embodiments, the cells expressing the P2X7 receptor binding domain can be cells which naturally express the P2X7 receptor or can be derived from a cell line that has been transformed with a genetic construct encoding the P2X7 receptor binding domain. When transformed cells are used, they are preferably transformed with a genetic construct expressing the entire P2X7 receptor, or a chimeric protein comprising at least the binding domain of the P2X7 receptor.

In another aspect, the present invention provides methods for screening compounds to identify therapeutic candidates for modulating bone mass and/or bone mineral density by modulating P2X7 receptor expression. In these methods, cells expressing an endogenous P2X7 receptor or cells transformed with a reporter gene operably joined to an endogenous or exogenous P2X7 receptor gene transcriptional control element are contacted with a compound to be tested. An increase or decrease in the expression of the endogenous P2X7 gene or the reporter gene indicates that the compound is a candidate for modulating bone mass and/or bone mineral density by increasing or decreasing P2X7 receptor expression *in vivo*.

In another aspect, the present invention provides for cell-based functional activity assays for screening compounds to identify therapeutic candidates for modulating bone mass and/or bone mineral density. In these methods, cells from a cell line transformed with a genetic construct including an exogenous transcriptional control element operably joined to a sequence encoding a P2X7 receptor are contacted with a compound to be tested. An increase or decrease in an indicator of P2X7 receptor activity in the cells indicates that the compound is a candidate for modulating bone mass and/or bone mineral density by increasing or decreasing P2X7 receptor activity *in vivo*. In such embodiments, the indicators may include, but are not limited to, increased cell permeability or increased intracellular accumulation of a macromolecule; increased IL-1α, IL-1β, or IL-18 post-translational processing in activated inflammatory cells; increased IL-6 production in inflammation-induced cells; L-selectin shedding; stress kinase activation; and lymphoproliferation. In some embodiments in which the cells are osteoclast progenitors, the indicator is differentiation into osteoclasts. In some embodiments in which the cells are osteoclasts, the indicator is increased resorption pit formation or increased apoptosis.

In any of the foregoing embodiments employing transformed cells, the cells can be derived from a transgenic non-human animal, or a descendant of such an animal, that has been transformed with a genetic construct encoding the P2X7 receptor, P2X7 receptor binding domain, or reporter gene.

In another aspect, the present invention provides animal-based assays for screening compounds to identify therapeutic candidates for modulating bone mass and/or bone mineral density, in which whole non-human animals are employed. Thus, in this aspect, a compound is administered to an animal, and an increase or decrease in an indicator of P2X7 receptor activity is detected in the animal. An increase in such an indicator indicates that the compound is a candidate for increasing bone mass and/or bone mineral density by increasing P2X7 receptor activity *in vivo*. A decrease in the indicator indicates that the compound is a candidate for decreasing bone mass and/or bone mineral density by decreasing P2X7 receptor activity *in vivo*.

In some embodiments, the animal is an animal which normally expresses the P2X7 receptor and which is normal or wild-type, with respect to the receptor. Alternatively, the animal is a transgenic non-human animal, or a descendant of such an animal, which has been transformed with genetic construct encoding a P2X7 receptor which is expressed in the animal.

Indicators of P2X7 receptor activity which can be detected in the foregoing embodiments include increased cell permeability or increased intracellular accumulation of a macromolecule; increased IL-1α, IL-1β, or IL-18 post-translational processing in activated inflammatory cells; increased IL-6 production in inflammation-induced cells; L-selectin shedding; stress kinase activation; and lymphoproliferation. In addition, the indicators include differentiation of osteoclast progenitors into osteoclasts, increased resorption pit formation, or increased apoptosis of osteoclasts. Such indicators are preferably assessed using fluid and/or biopsy samples from the animal. In addition, the indicators include phenotypic changes such as bone mineral density, bone mineral content, trabecular bone number, trabecular bone separation, bone mineral area, bone volume, bone thickness, and bone circumference.

In another aspect, the present invention provides for methods for screening compounds to identify therapeutic candidates for modulating bone mass and/or bone mineral density in which a compound is administered to an animal. In these embodiments, the animal is a transgenic non-human animal, or a descendant of such an animal, which has been transformed with an exogenous reporter gene which is operably joined to an exogenous or endogenous P2X7 receptor gene transcriptional control element. An increase or decrease in the expression of the reporter gene in the animal indicates that the compound is a candidate for increasing or decreasing bone mass and/or bone mineral density by increasing or decreasing P2X7 receptor expression *in vivo*.

In another aspect, the present invention provides cell-free binding assays for screening compounds to identify therapeutic candidates for modulating bone mass and/or bone mineral density. In these methods, binding assays are performed including at least a binding domain of a P2X7 receptor, but the receptor need not be associated with a cell. Thus, in some embodiments, a compound is contacted with at least a P2X7 receptor binding domain under conditions which, but for the presence of the compound, permit binding of said P2X7 receptor binding domain to a known P2X7 receptor ligand *in vivo*. Binding, if any, between the P2X7 receptor binding domain and compound is detected. In such assays, binding between the P2X7 receptor binding domain and the compound indicates that the compound is a candidate for modulating bone mass and/or bone mineral density by interacting with a P2X7 receptor *in vivo.* In other embodiments, a compound is contacted with at least a binding domain of a P2X7 receptor in the presence of a known P2X7 receptor ligand, under conditions which, but for the presence of the compound, permit binding of the P2X7 receptor binding domain to the P2X7 receptor ligand *in vivo*. Binding, if any, between the P2X7 receptor binding domain and either the compound or the P2X7 receptor ligand is detected. In such assays, binding between the P2X7 receptor binding domain and the compound, or the detection of decreased binding between the P2X7 receptor binding domain and the P2X7 receptor ligand, indicates that the compound is a candidate for modulating bone mass and/or bone mineral density by interacting with a P2X7 receptor *in vivo*.

In any of the preceding embodiments, in order to facilitate detection of binding amongst the compound, the known ligand and/or the P2X7 receptor binding domain, either the compound or the known P2X7 receptor ligand are preferably labeled with a detectable label. After contacting the labeled element with the P2X7 receptor binding domain on the cell surface, any unbound labeled element is washed away, and the labeled element bound to the P2X7 receptor binding domain is detected. Alternatively, if a scintillation proximity assay is employed, the excess labeled element need not be washed away prior to detection. Labels which are used in the foregoing binding assays include radioisotopes, fluorescent labels, chemiluminescent labels, enzymatic domains, specific binding elements, and metal atoms.

In preferred embodiments of the foregoing binding assays, one of the non-labeled binding elements is immobilized. Thus, in some embodiments, the compound is labeled and the P2X7 receptor binding domain is immobilized on a substrate. In other embodiments, the P2X7 receptor ligand is labeled and the P2X7 receptor binding domain is immobilized on a substrate. In other embodiments, the P2X7 receptor binding domain is labeled and the compound is immobilized on a substrate. In other embodiments, the P2X7 receptor ligand is labeled and the compound is immobilized on a substrate. In other embodiments, the compound is labeled and the P2X7 receptor ligand is immobilized on a substrate. Finally, in other embodiments, the P2X7 receptor binding domain is labeled and the P2X7 receptor ligand is immobilized on a substrate. Substrates appropriate for the immobilized elements include affinity columns, slides, wells, particles and/or microparticles.

In another aspect, the present invention provides methods for screening compounds to identify therapeutic candidates for modulating bone mass and/or bone mineral density, in which aggregation assays are employed. In these methods, a compound is immobilized on a first particle, and at least a binding domain of a P2X7 receptor is immobilized on a second particle. The particles are contacted under conditions which, but for the presence of the compound, permit binding of said P2X7 receptor binding domain to a P2X7 receptor ligand. Binding between the P2X7 receptor binding domain and the compound is detected by detecting aggregation of the particles. Aggregation indicates that the compound is a candidate for modulating bone mass and/or bone mineral density by interacting with a P2X7 receptor *in vivo.*

In another aspect, the present invention provides methods for increasing bone mass and/or bone mineral density in a mammal in need of such treatment by administering an amount of an agonist of a P2X7 receptor effective to increase bone mass and/or bone mineral density in the mammal. In another aspect, the present invention provides methods for treating a mammal having a skeletal disorder characterized by decreased bone mass and/or bone mineral density by administering an amount of an agonist of a P2X7 receptor effective to increase bone mass and/or bone mineral density in the mammal. In some embodiments of these methods, the mammal is afflicted with, or at substantial risk of, a disorder such as osteoporosis, periodontitis, orthopedic osteolysis, inflammatory bone diseases, or bone fracture. The P2X7 receptor agonist may be administered systemically or locally.

Those skilled in the art will fully understand the terms used herein in the description and the appendant claims to describe the present invention. Nonetheless, unless otherwise provided herein, the following terms are as described immediately below.

As used herein, the term "P2X7 receptor" refers to the human receptor described by Rassendren et al. (1997), *J. Biol. Chem.* 272:5482-5486, as well as any human allelic variants and any mammalian homologs having P2X7 receptor activity and their allelic variants (see also GenBank Accession No. Y09561 and GenBank Accession Nos. Y12851-Y12855). As used herein, the term "homolog" means a protein which is evolutionarily related to and shares substantial structural and functional similarity with a reference protein in a different species (e.g., human and rat P2X7 receptors).

As used herein, the term "P2X7 activity" means any one or more of the following: increased cell permeability; increased intracellular accumulation of a macromolecule; increased IL-1α, IL-1β, or IL-18 post-translational processing in activated inflammatory cells; increased IL-6 production in inflammation-induced cells; L-selectin shedding; stress kinase activation; lymphoproliferation; differentiation of osteoclast progenitors into osteoclasts; increased resorption pit formation by osteoclasts; or increased apoptosis of osteoclasts. These activities are known to be stimulated by ATP. In the methods of the invention, however, these activities are stimulated by test compounds with or without the presence of a P2X7 receptor ligand.

As used herein, the term "binding domain of a P2X7 receptor" or "P2X7 receptor binding domain" means that portion of the P2X7 receptor protein which binds naturally-occurring, non-immunoglobulin ligands *in vivo*. As used herein, the binding domain of the P2X7 receptor includes at least the extracellular domain of the molecule, corresponding to approximately amino acid residues 42-327 of GenBank Accession No. Y09561, and preferably includes the first transmembrane domain, corresponding to approximately residues 23-41 of GenBank Accession No. Y09561.

As used herein, the term "P2X7 receptor ligand" means any molecule which binds to the P2X7 receptor and acts as an agonist. A number of P2X7 receptor agonists have already been identified (see Burnstock and Williams (2000), *J. Pharmacol. Exp. Ther.* 295:862-869). For example, known agonists of the P2X7 receptor include adenosine triphosphate (ATP), 2'-O-(4-benzoyl)-benzoyl-ATP, 3'-O-(4-benzoyl)-benzoyl-ATP (Bz-ATP), and 1-α,β-methylene ATP (α,β-meATP). As will be apparent to one of ordinary skill in the art, chemical variants, derivatives and analogs of these compounds, as well as chemical variants, derivatives and analogs of the natural P2X7 receptor ligand ATP, are also candidates for additional agonists to be included as P2X7 receptor ligands.

As used herein with respect to assays, the phrase "conditions which permit binding of a P2X7 receptor binding domain to a P2X7 receptor ligand" means conditions of temperature, pressure, pH, ionic concentrations and osmolality in an assay medium which are the same as, or sufficiently similar to, physiological conditions, such that the P2X7 receptor and a P2X7 receptor ligand are capable of binding to substantially the same degree that they bind *in vivo*. Such conditions assume that the assay medium is essentially free of effective inhibitors or competitors of receptor-ligand binding except, potentially, for the compound being screened.

As used herein, the phrase "genetic construct encoding a P2X7 receptor binding domain" means a recombinant DNA, RNA, or nucleic acid analog molecule which includes a genetic sequence encoding, or which is complementary to a genetic sequence encoding, the amino acid sequence of at least the binding domain of the P2X7 receptor, and which is capable of being expressed in a cell which has been transformed with the construct. The construct may express the P2X7 protein transiently, or may stably integrate into the genome of the cell and express the protein inducibly or constitutively. As used herein, a "nucleic acid analog" means a molecule having sufficient structural and functional similarity to a nucleic acid to direct forward or reverse transcription of complementary nucleic acids, or to direct translation of an encoded polypeptide within a living cell.

As used herein, with respect to genetic engineering, the term "transform" means to introduce into a cell or an organism an exogenous nucleic acid or nucleic acid analog which encodes a polypeptide sequence which is expressed in that cell or organism, and/or is integrated into the genome of that cell or organism so as to affect the expression of a genetic locus. The term "transform" is used to embrace all of the various methods of introducing such nucleic acids or nucleic acid analogs, including, but not limited to the methods referred to in the art as transformation, transfection, transduction, electroporation, ballistic injection, and the like.

As used herein, the term "exogenous" means, with respect to a reference genetic sequence or genome, that a second genetic sequence does not naturally occur contiguous to the reference sequence or within the reference genome.

As used herein, the term "reporter gene" means any genetic sequence which, when expressed, has a biochemical or phenotypic effect which is detectable.

As used herein, the phrase *"A* is contacted with *B"* means that *A* and *B* are brought into sufficient physical proximity to interact at the molecular level, as by mixing *A* and *B* together in a solution, or pouring a solution of *A* over *B* on substrate. As used herein, the phrase "*A* is contacted with *B*" is intended to be equivalent to *"B* is contacted with *A*" and is not intended to imply that either element is fixed relative to the other, or that either element is moved relative to the other.

As used herein, the term "therapeutically effective amount" means the total amount of each active component of a pharmaceutical composition or method that is sufficient to show a meaningful patient benefit, *i.e.,* reducing the size of a non-union fracture or bone lesion, promoting bone growth or healing, increasing bone mass, or increasing bone mineral density. When applied to an individual active ingredient, administered alone, the term refers to that ingredient alone. When applied to a combination, the term refers to combined amounts of the active ingredients that result in the therapeutic effect, whether administered in combination, serially or simultaneously.

As used herein, the terms "modulate" or "affect" mean to either increase or decrease. As used herein, the terms "increase" and "decrease" mean, respectively, statistically significantly increase (*i.e.,* p < 0.1) and statistically significantly decrease *(i.e.,* p < 0.1).

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., Basic Methods in Molecular Biology, Elsevir Sciences Publishing, Inc., New York, NY,1986; Hames et al., Nucleic Acid Hybridization, IL Press, 1985; Molecular Cloning, Sambrook et al., Current Protocols in Molecular Biology, Eds. Ausubel et al., John Wiley and Sons; Current Protocols in Human Genetics, Eds. Dracopoli et al., John Wiley and Sons; Current Protocols in Protein Science, Eds. John E. Coligan et al., John Wiley and Sons; and Current Protocols in Immunology, Eds. John E. Coligan et al., John Wiley and Sons). All publications mentioned herein are incorporated by reference in their entireties.

### DESCRIPTION OF FIGURES

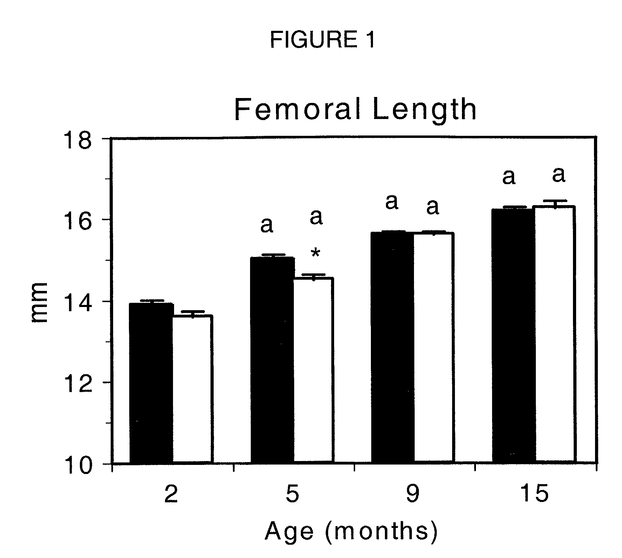

Figure 1 shows Femoral length in P2X7RKO male mice (white bars) and WT controls (black bars) at 2, 5, 9, and 15 months of age, *: p < 0.05 vs. age-matched WT controls. a: p < 0.05 vs. the same phenotype at the previous age group (* indicates statistically significant difference).

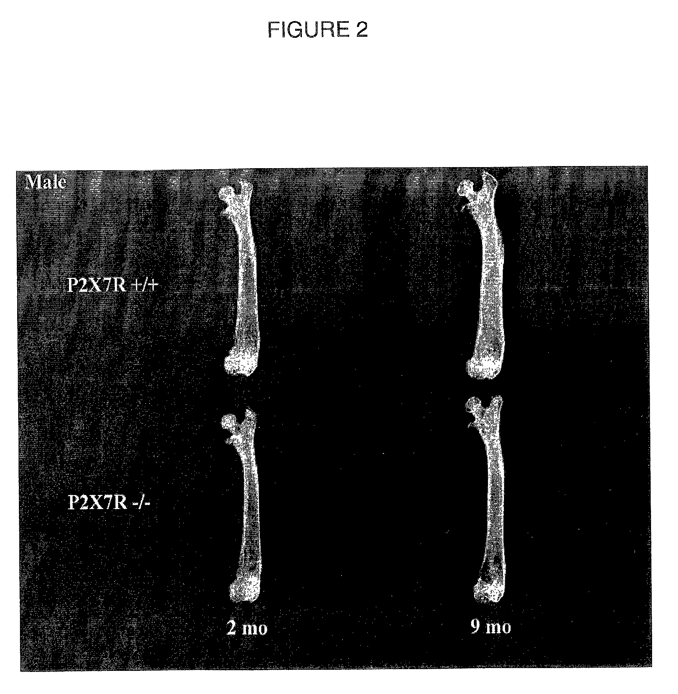

Figure 2 shows X-ray images of femora from P2X7R -/- male mice as compared with those from the wild-type littermate, age- and gender matched controls (P2X7R +/+) at 2-and 9-month-old. P2X7R -/- mice had similar femoral length but smaller diameter as compared with P2X7R +/+ mice.

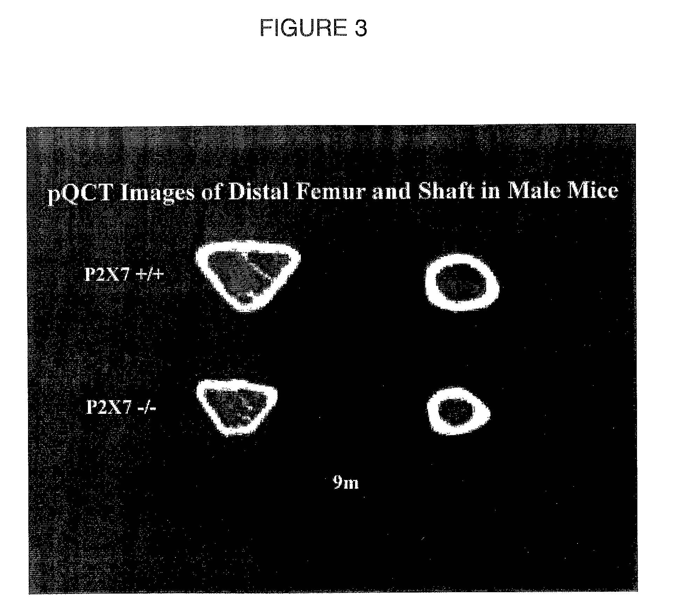

Figure 3 shows PACT images of distal femoral metaphysis (left panel) and femoral shafts (right panel) from P2X7R -/- male mice as compared with those from the wild-type littermate, age- and gender matched controls (P2X7R +/+) at 9-month-old. P2X7R -/mice had smaller bone at both sites as compared with P2X7R +/+ mice.

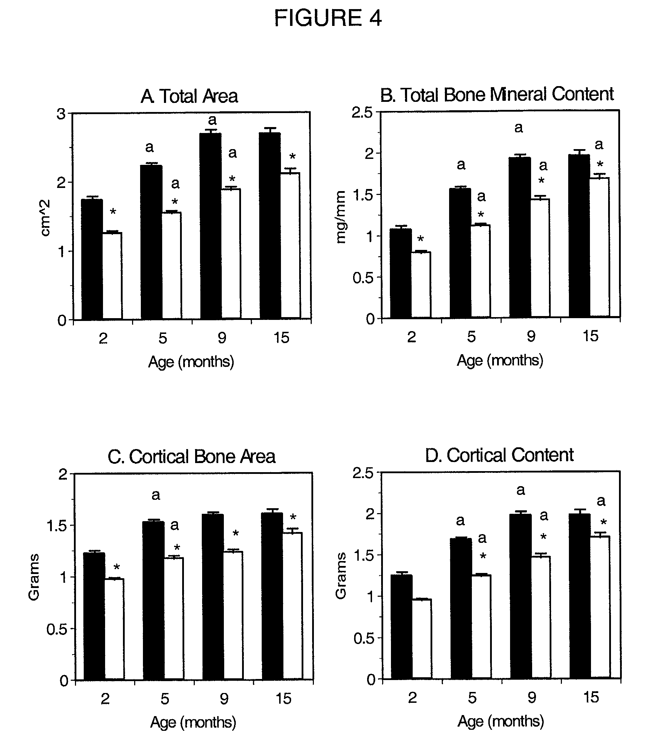

Figure 4 shows Total bone area (A), total bone mineral content (B), trabecular content (C) and cortical content (D) of distal femoral metaphysis determined by pQCT in P2X7RKO male mice (white bars) and WT controls (black bars) at 2, 5, 9, and 15 months of age, *: p < 0.05 vs. age-matched WT controls. a: p < 0.05 vs. the same phenotype at the previous age group.

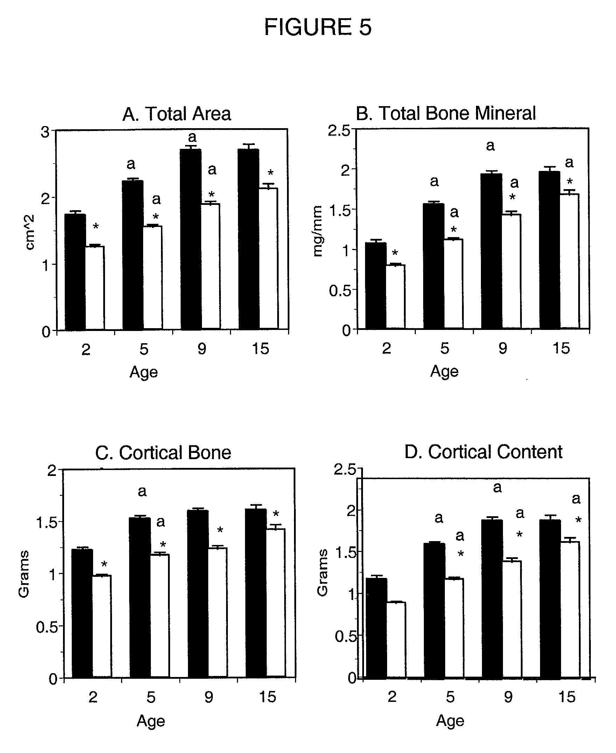

Figure 5 shows Total bone area (A), total bone mineral content (B), cortical bone area (C) and cortical content (D) of femoral shafts determined by pQCT in P2X7RKO male mice (white bars) and WT controls (black bars) at 2, 5, 9, and 15 months of age, *: p < 0.05 vs. age-matched WT controls. a: p < 0.05 vs. the same phenotype at the previous age group.

### DETAILED DESCRIPTION

The present invention depends, in part, upon the discovery and characterization of the role of the P2X7 receptor and its activities *in vivo.* Specifically, the present invention is based, in part, upon the discovery that the P2X7 receptor plays an important role in bone mass augmentation and maintenance, including the regulation of cancellous and cortical bone mass and the development of cancellous bone architecture. Accordingly, the present invention provides methods for screening compounds to identify therapeutic candidates for modulating bone mass and/or bone mineral density, as well as methods for treating skeletal disorders characterized by decreased bone mass and/or bone mineral density using of agonists of the P2X7 receptor.

### 1. Candidates for P2X7 Receptor Agonists and Antagonists

Examples of classes of compounds that can be screened to identify P2X7 receptor agonists or antagonists include, but are not limited to, nucleic acids (*e.g.*, DNA and RNA), carbohydrates, lipids, proteins, peptides, peptidomimetics, and small molecules.

Candidate compounds can be identified from libraries of agents that are obtained using any of numerous approaches known in the art, including both libraries of naturally-occurring compounds and libraries of synthetic compounds produced by combinatorial chemistry.

Examples of methods for the synthesis of molecular libraries can be found in the art, for example in: U.S. Patent No. 5,738,996; U.S. Patent No. 5,807,683; DeWitt et al. (1993), *Proc. Natl. Acad. Sci. (USA)* 90:6909; Erb et al. (1994), *Proc. Natl. Acad. Sci. (USA)* 91:11422; Zuckermann et al. (1994), *J. Med. Chem.* 37:2678; Cho et al. (1993), *Science* 261:1303; Carrell et al. (1994), *Angew. Chem. Int. Ed. Engl.* 33:2059; Carell et al. (1994), *Angew. Chem. Int. Ed. Engl.* 33:2061; Gallop et al. (1994), *J. Med. Chem.* 37:1233; and Lam (1997), *Anticancer Drug Des.* 12:145.

Libraries of agents can be presented in solution *(e.g.,* Houghten (1992), *Bio*/*Techniques* 13:412-421), or on beads (Lam (1991), *Nature* 354:82-84), chips (Fodor (1993), *Nature* 364:555-556), bacteria (U.S. Patent No. 5,223,409), spores (U.S. Patent No. 5,571,698; U.S. Patent No. 5,403,484; and U.S. Patent No. 5,223,409), plasmids (Cull et al. (1992), *Proc. Natl. Acad. Sci. (USA)* 89:1865-1869) or phage (Scott and Smith (1990), *Science* 249:386-390; Devlin (1990), *Science* 249:404-406; Cwirla et al. (1990), *Proc. Natl. Acad. Sci.* (*USA*) 87:6378-6382; and Felici (1991), *J. Mol. Biol.* 222:301-310).

### 2. Cell-Based Functional Assays

The present invention provides several methods for screening compounds to identify therapeutic candidates for modulating bone mass and/or bone mineral density, in which living cells which express at least the binding domain of the P2X7 receptor are employed. These methods may employ cells which naturally express the P2X7 receptor or cells which have been genetically engineered to cause or enhance P2X7 receptor expression. The cells can be mammalian (*e.g.*, human, monkey, mouse, rat, canine, hamster, rabbit, goat), or can be other eukaryotic *(e.g.,* insect or yeast) or prokaryotic (*e.g.*, bacterial) cells which have been genetically engineered to express a human or other mammalian P2X7 receptor. The cells can be obtained from immortalized cell lines, cell cultures, or primary cell preparations, or can be obtained directly from animals *(e.g.,* a human or other mammal, or a non-human transgenic animal). In preferred embodiments where binding to a P2X7 receptor is assessed, the cells are human, mouse, rat, or hamster cells expressing at least the binding domain of the human P2X7 receptor. In most preferred embodiments, the cells are mammalian, more preferably, human or murine, and/or are macrophages or osteoclasts which express the P2X7 receptor endogenously. Because the C-terminus of the P2X7 receptor is believed to be involved in channel or pore formation, when the cells are transformed with a genetic construct, it is preferred that the construct encode the entire P2X7 receptor, or that sequences encode a chimeric protein including the binding domain joined to a C-terminal sequence which functions equivalently to the natural P2X7 C-terminal sequence (see, *e.g.,* Rassendren et al. (1997), *J. Biol. Chem.* 272:5482-5486, reporting the production of chimeric human-rat P2X7 receptors).

In a first aspect, the invention provides methods for determining the ability of a compound to increase or decrease an indicator of P2X7 receptor activity in a living cell. In these methods, a compound is contacted with cells from a cell line expressing the P2X7 receptor on their cell surfaces. The contacting step is conducted under conditions which, but for the presence of the compound, would permit the binding of the P2X7 receptor to a P2X7 receptor ligand. Thus, for example, the contacting step can be carried out in a physiologically acceptable buffer solution or in the cell culture medium in which the cells are grown. Preferably, the conditions are chosen to be substantially duplicative of *in vivo* conditions.

After contacting the cells with a test compound, an indicator of P2X7 receptor activity is measured. An increase or decrease in the indicator indicates that the compound is a candidate for modulating bone mass and/or bone mineral density by interacting with a P2X7 receptor *in vivo.* Appropriate indicators of P2X7 receptor activity are discussed below.

In some embodiments of cell-based screening methods when the methods are used to identify P2X7 receptor agonists, the test compound is contacted with the P2X7 receptor without the addition of any known P2X7 receptor ligand to the assay medium. Therefore, in these embodiments, any increase in the indicator of P2X7 receptor activity indicates that the compound is a candidate for an agonist of the P2X7 receptor.

In other embodiments, a known P2X7 receptor ligand, such as those described above, is added to the assay medium in addition to the compound to be tested. In these embodiments, an increase in the indicator of P2X7 receptor activity indicates that the compound is a candidate for either an agonist of the P2X7 receptor or an enhancer of the agonist activity of the known ligand. Conversely, a decrease in the indicator indicates that the compound is a candidate for either an antagonist of the P2X7 receptor or an inhibitor of the agonist activity of the known ligand. In preferred embodiments, the known ligand of the P2X7 receptor which is added to the assay medium is ATP.

### 3. Cell-Based Binding Assays

In another aspect, the present invention provides methods for screening compounds to identify therapeutic candidates for modulating bone mass and/or bone mineral density, by determining the ability of a compound to bind to a P2X7 receptor binding domain. In these methods, a compound is contacted with at least the binding domain of the P2X7 receptor on the surface of cells from a cell line expressing the binding domain of the P2X7 receptor on their surfaces. The contacting step is conducted under conditions which, but for the presence of the compound, would permit the binding of the P2X7 receptor binding domain to a P2X7 receptor ligand. Binding, if any, between the P2X7 receptor binding domain and the compound is detected. Binding between the P2X7 receptor binding domain and the compound indicates that the compound is a candidate for modulating bone mass and/or bone mineral density by interacting with a P2X7 receptor *in vivo*.

In some embodiments, the compound is contacted with the P2X7 receptor binding domain without the addition of a known P2X7 receptor ligand to the assay. In these embodiments, binding to the P2X7 receptor binding domain indicates that the compound is a candidate for interacting with the P2X7 receptor. In other embodiments, the compound is added in the presence of a known P2X7 receptor ligand. In these embodiments, either binding of the compound to the P2X7 receptor binding domain, or a decrease in the binding of the known ligand to the P2X7 receptor binding domain, indicates that the compound is a candidate for interacting with the P2X7 receptor.

In any of the preceding embodiments, in order to facilitate detection of binding amongst the compound, the known ligand and/or the P2X7 receptor binding domain, either the compound or the known P2X7 receptor ligand are preferably labeled with a detectable label. After contacting the labeled element with the P2X7 receptor binding domain on the cell surface, any unbound labeled element is washed away, and the labeled element bound to the P2X7 receptor binding domain is detected. The labels which can be used in such assays include, but are not limited to, radioisotopes (*e.g.*, ³²P, ³⁵S), fluorescent labels (*e.g.,* fluorescein isothiocyanate, rhodamine, phycoerythrin, phycocyanin, allophycocyanin, and fluorescamine), chemiluminescent labels (*e.g.*, luciferin, luminol, isoluminol, aromatic acridinium esters, imidazoles, and the oxalate esters), enzymatic domains *(e.g.,* luciferase, alkaline phosphatase, β-galactosidase, glucose-6-phosphate dehydrogenase, maleate dehydrogenase, glucose oxidase, and peroxidase), specific binding elements (*e.g*., *c-myc* epitope, biotin or (strept)avidin, Protein A, lectin, immunoglobulins), and metal atoms *(e.g.,* Au).

In any of the above-described embodiments, the cells expressing the P2X7 receptor binding domain can be cells which naturally express the P2X7 receptor or can be derived from a cell line that has been transformed with a genetic construct encoding the P2X7 receptor binding domain. When transformed cells are used, they can be transformed with a genetic construct expressing the entire P2X7 receptor, or a chimeric protein comprising at least the binding domain of the P2X7 receptor.

### 4. Animal-Based Assays

In another aspect, the present invention provides methods for screening compounds to identify therapeutic candidates for modulating bone mass and/or bone mineral density, in which whole non-human animals are employed. Thus, in this aspect, a compound is administered to an animal, and an increase or decrease in an indicator of P2X7 receptor activity is detected in the animal. An increase in such an indicator indicates that the compound is a candidate for increasing bone mass and/or bone mineral density by increasing P2X7 receptor activity *in vivo*. A decrease in the indicator indicates that the compound is a candidate for decreasing bone mass and/or bone mineral density by decreasing P2X7 receptor activity *in vivo.* Appropriate indicators of P2X7 receptor activity are discussed below.

In some embodiments, the animal is an animal which normally expresses the P2X7 receptor and which is normal or wild-type, with respect to the receptor. Alternatively, the animal is a transgenic non-human animal, or a descendant of such an animal, which has been transformed with genetic construct encoding a P2X7 receptor which is expressed in the animal, preferably in tissues which express an endogenous P2X7 receptor. Transformation vectors can be produced and used to transform blastocysts or embryonic stem cells, and these transformed cells can then be used to produce transgenic animals by methods known in the art (see, *e.g.,* Solle et al. (2001), *J. Biol. Chem.* 276:125-132).

Cells from a non-human transgenic animal can be isolated from tissue or organs using techniques known to those of skill in the art and used in the above-described cell-based activity assays and cell-based binding assays. Such cells can also be used as a source of P2X7 receptor proteins for cell-free binding assays, as discussed below, the generation of antibodies, and the like. For example, immune cells can be collected or isolated from blood, or secondary lymphoid organs of the subject, such as, but not limited to, lymph nodes, tonsils, the spleen, Peyer's patch of the intestine, and bone marrow, by any of the methods known in the art (see, *e.g.,* Current Protocols in Immunology, Green Publishing Associates, New York, NY, (1991), p. 21). Immune cells obtained from such sources typically comprise predominantly recirculating lymphocytes and macrophages at various stages of differentiation and maturation. Optionally, standard techniques, such as morphological observation and immunochemical staining, can be used, if desired, to verify the presence of the desired cells (*e.g.*, dendritic cells, T cells, and macrophages). The immune cells used in the cell based methods of the invention can be collected by standard techniques. For example, a syringe can be used to withdraw blood, which is subjected to Ficoll-Hypaque (Pharmacia) gradient centrifugation to separate cells. Blood, anticoagulated with preservative-free heparin, usually yields 0.5-1.0 x 10⁶ lymphocytes/ml. Separated blood cells (*e.g.*, monocytes) can be used in the present methods. In preferred embodiments, the immune cells used are purified white blood cells comprising lymphocytes and macrophages.

Monocyte-derived macrophages can be isolated from immune cells by allowing the cells to adhere to culture flasks for 1-2 hours, washing away nonadherent cells and culturing adherent cells for 7-12 days in an appropriate medium (*e.g.*, RMPI 1640 supplemented with 20% human serum, 2 mM glutamine, 5 mM HEPES (4-(2-hydroxyethyl)-1-piperazine-ethane-sulphonic acid), and 100 µg/ml streptomycin) plus interferon-y (IFN-y) (see, *e.g.,* Blanchard et al. (1995), *J. Cell. Biochem.* 57:452-464; and Blanchard et al. (1991), J. *Immunol*. 147:2579-2585).

### 5. Indicators of P2X7 Activity

In any of the cell-based or animal-based methods described herein, the interaction between the test compound or ligand and the receptor can be determined by measuring an indicator of P2X7 receptor activity. These indicators are known to be associated with ATP-stimulation of the P2X7 receptor. Therefore, in the methods of the invention, these indicators are assayed either with and without concomitant stimulation by a P2X7 receptor ligand. These indicators include, but are not limited to, increased cell permeability or increased intracellular accumulation of a macromolecule; increased IL-1α, IL-1β, or IL-18 post-translational processing in activated inflammatory cells as an indirect increase IL-6 production in inflammation-induced cells; L-selectin shedding (see, *e.g.,* Gu et al. (1998), *Blood* 92:946-951); stress kinase activation (se, *e.g.,* Humphreys et al. (2000), *J*. *Biol. Chem.* 275(35):26792-26798); and lymphoproliferation.

In embodiments in which the cells used are osteoclast progenitors, the indicator can be differentiation into an osteoclast, and in embodiments in which the cells are osteoclasts, the indicator can be increased resorption pit formation (see, *e.g.,* Morrison et al. (1998), *J. Physiol*. 511:495-500) or increased apoptosis (see, *e.g.*, Hogquist et al. (1991), *Proc. Natl. Acad*. *Sci*. *(USA)* 88:8485-8489; Modderman et al. (1994), *Calcif. Tissue Int*. 55(2):141-150). Such indicators are assessed using cells cultured *in vitro,* or using fluid and/or biopsy samples from the animal.

Methods of measuring such indicators of P2X7 activity are described herein and in the literature. For examples, changes in cell permeability can be measured by cellular electrophysiological changes and/or the cellular accumulation of macromolecules such as dyes (see, *e.g.,* Michel et al. (1998), *Br. J. Pharmacol.* 124:1194-1201; Virginio et al. (1999), J. *Physiol.* 519:335-346; and the Examples below). Changes in post-translational processing and production of interleukins can be measured using immunoprecipitation and/or ELISA assays (see, *e.g.,* Solle et al. (2001), *J*. *Biol. Chem.* 276:125-132; and the Examples below). Changes in lymphoproliferation can be measured by standard cell counting techniques (see, *e.g.,* Baricordi et al. (1999), *J. Biol. Chem.* 274:33206-33208; Chen (1996), *Oncogene* 13:1395-403; Jeoung (1995), *J. Biol. Chem.* 270:18367-73).

In addition, in any of the animal-based methods described herein, indicators of P2X7 receptor activity may include phenotypic changes in parameters such as, but not limited to, bone mineral density, bone mineral content, trabecular bone number, trabecular bone separation, bone mineral area, bone volume, bone thickness, and bone circumference. In particular, indicators of increased P2X7 activity may include higher volumetric cortical-subcortical bone mineral content, higher volumetric cortical-subcortical bone mineral density, higher volumetric cortical bone mineral content, higher volumetric cortical bone mineral density, higher trabecular bone volume, higher trabecular bone thickness, higher trabecular bone number, and/or lower trabecular bone separation at the distal femoral metaphysis; or higher volumetric cortical-subcortical bone mineral content, higher volumetric cortical bone mineral content, higher volumetric cortical bone mineral area, higher volumetric trabecular bone mineral content, higher volumetric total bone mineral content, higher volumetric total bone mineral density, higher periosteal circumference, and/or higher endocortical circumference at the femoral shafts.

### 6. Cell-Free Binding Assays

In another aspect, the present invention provides cell-free binding assays for screening compounds to identify therapeutic candidates for modulating bone mass and/or bone mineral density. In these methods, binding assays are performed using at least a binding domain of a P2X7 receptor, but the receptor protein need not be associated with a cell. Thus, in some embodiments, a compound is contacted with at least a P2X7 receptor binding domain under conditions which, but for the presence of the compound, permit binding of said P2X7 receptor binding domain to a known P2X7 receptor ligand *in vivo*. Binding, if any, between the P2X7 receptor binding domain and compound is then detected. In such assays, binding between the P2X7 receptor binding domain and the compound indicates that the compound is a candidate for modulating bone mass and/or bone mineral density by interacting with a P2X7 receptor *in vivo*. In other embodiments, a compound is contacted with at least a binding domain of a P2X7 receptor in the presence of a known P2X7 receptor ligand, under conditions which, but for the presence of the compound, permit binding of the P2X7 receptor binding domain to the P2X7 receptor ligand. Binding, if any, between the P2X7 receptor binding domain and either the compound or the P2X7 receptor ligand is then detected. In such assays, binding between the P2X7 receptor binding domain and the compound, or the detection of increased or decreased binding between the P2X7 receptor binding domain and the P2X7 receptor ligand, indicates that the compound is a candidate for modulating bone mass and/or bone mineral density by interacting with a P2X7 receptor *in vivo*.

In any of the preceding embodiments, in order to facilitate detection of binding amongst the compound, the known ligand and/or the P2X7 receptor binding domain, either the compound or the known P2X7 receptor ligand are preferably labeled with a detectable label. After contacting the labeled element with the P2X7 receptor binding domain on the cell surface, any unbound labeled element is washed away, and the labeled element bound to the P2X7 receptor binding domain is detected. Alternatively, if a scintillation proximity assay is employed, the excess labeled element need not be washed away prior to detection. Labels which can be used in the foregoing binding assays include, but are not limited to, radioisotopes, fluorescent labels, chemiluminescent labels, enzymatic domains, specific binding elements, and metal atoms, as described above.

In preferred embodiments of the foregoing binding assays, one of the non-labeled binding elements is immobilized. Thus, in some embodiments, the compound is labeled and the P2X7 receptor binding domain is immobilized on a substrate. In other embodiments, the P2X7 receptor ligand is labeled and the P2X7 receptor binding domain is immobilized on a substrate. In other embodiments, the P2X7 receptor binding domain is labeled and the compound is immobilized on a substrate. In other embodiments, the P2X7 receptor ligand is labeled and the compound is immobilized on a substrate. In other embodiments, the compound is labeled and the P2X7 receptor ligand is immobilized on a substrate. Finally, in other embodiments, the P2X7 receptor binding domain is labeled and the P2X7 receptor ligand is immobilized on a substrate. Substrates appropriate for immobilizing these elements include affinity columns, slides, wells, particles and/or microparticles.

In another aspect, methods are provided for screening compounds to identify therapeutic candidates for modulating bone mass and/or bone mineral density, in which aggregation assays are employed. In these methods, a compound is immobilized on a first particle and at least a binding domain of a P2X7 receptor is immobilized on a second particle. The particles are contacted under conditions which, but for the presence of the compound, permit binding of said P2X7 receptor binding domain to a P2X7 receptor ligand. Binding between the P2X7 receptor binding domain and the compound is detected by detecting aggregation of the particles. Aggregation indicates that the compound is a candidate for modulating bone mass and/or bone mineral density by interacting with a P2X7 receptor *in vivo*. Aggregation is measured using standard techniques, including turbidity measurements. Particles suitable for use in the invention include latex, polylactic acid (PLA), polyglycolic acid (PGA), poly(co-lactic- glycolic) acid (PLGA), poly(ethylene glycol) (PEG), and similar microspheres, having size ranges of from 1 µm-1000 µm.

### 7. Assays for Modulators of P2X7 Expression

In another aspect, the present invention provides methods for screening compounds which may modulate the expression of the P2X7 receptor by modulating the transcription of the P2X7 receptor gene. Such compounds are therapeutic candidates for modulating bone mass and/or bone mineral density.

In one embodiment of these methods, cells that express an endogenous P2X7 receptor are used and expression of the sequence encoding the P2X7 receptor is measured by detecting P2X7 mRNA or protein levels using standard methods known in the art. Alternatively, cells are employed from a cell line transformed with an exogenous reporter gene operably joined to an exogenous or endogenous P2X7 receptor gene transcriptional control element. These cells are contacted with a compound to be tested, and an increase or decrease in the expression of the reporter gene indicates that the compound is a candidate for modulating bone mass and/or bone mineral density by increasing or decreasing P2X7 receptor expression *in vivo*. Non-limiting examples of reporter genes which can be used in these methods include horseradish peroxidase, β-glucuronidase (GUS), β-galactosidase, luciferase, and chloramphenicol acetyltransferase (CAT) (see, *e.g.,* Jefferson (1987), *Plant Mol. Biol. Rep.* 5:387; Teeri et al. (1989), *EMBO J*. 8:343; Koncz et al. (1987), *Proc. Natl. Acad. Sci. (USA)* 84:131; De Block et al. (1984), *EMBO J.* 3:1681). If endogenous P2X7 receptor transcriptional control elements are to be operably linked to the reporter gene, a variety of transformation vectors, including but not limited to, homologous recombination vectors or site-specific insertion vectors can be produced according to methods known in the art. For example, a genetic construct for homologous recombination can be prepared which includes the coding sequence of the reporter gene flanked by sufficient P2X7 receptor gene sequences to permit homologous recombination and substitution of the reporter gene sequences for P2X7 receptor gene such that the expression of the reporter gene will be regulated by the P2X7 receptor gene transcriptional control elements. Alternatively, exogenous P2X7 receptor gene transcriptional control sequences can be operably joined to an exogenous reporter gene in a genetic construct by methods well known in the art. These constructs are designed to insert into the genome of cells (*e.g.,* by homologous recombination, site-specific insertion, or random insertion) or to exist extra-chromosomally (*e.g.*, as a plasmid).

Expression-based assays can also be conducted on animals which express the P2X7 receptor endogenously or transgenically. In one embodiment of these methods, an animal is employed which is a transgenic non-human animal, or a descendant of such an animal, which has been transformed with an exogenous reporter gene operably joined to an exogenous or endogenous P2X7 receptor gene transcriptional control element. An increase or decrease in the expression of the P2X7 receptor gene or the reporter gene in the animal indicates that the compound is a candidate for increasing or decreasing bone mass and/or bone mineral density by increasing or decreasing P2X7 receptor expression *in vivo.* The transformation vectors are produced, as described above, and used to transform blastocysts or embryonic stem cells, and these transformed cells are then used to produce transgenic animals by methods known in the art (see, *e.g.,* Solle et al. (2001), *J. Biol. Chem.* 276:125-132).

### 8. Genetic Constructs

To produce the transformed cells and/or transgenic non-human animals of the invention, a variety of standard recombinant genetic constructs can be employed.

The genetic constructs are introduced into target cells as structural components of any of a wide range of vectors that can be specifically or nonspecifically inserted into the target cell genome. Suitable expression vectors include bacterial, plasmid, yeast, and viral vectors. The viral vectors include, but are not limited to, herpes simplex virus vectors, adenovirus vectors, adeno-associated virus vectors, retroviral vectors, lentiviral vectors, pseudorabies virus vectors, alpha-herpes virus vectors, and the like. A thorough review of viral vectors, particularly viral vectors suitable for modifying nonreplicating cells, and how to use such vectors in conjunction with the expression of polynucleotides of interest can be found in *Viral Vectors: Gene Therapy and Neuroscience Applications,* Caplitt and Loewy, eds., Academic Press, San Diego, 1995. Retroviral vectors can be used in conjunction with retroviral packaging cell lines such as those described in U.S. Patent No. 5,449,614. Where non-murine mammalian cells are used as target cells for genetic modification, amphotropic or pantropic packaging cell lines can be used to package suitable vectors (Ory et al. (1996), *Proc. Natl. Acad. Sci. (USA)* 93:11400-11406). Representative retroviral vectors are described, for example, in U.S. Patent No. 5,521,076.

Genetic constructs intended to cause or enhance expression of the P2X7 receptor binding domain will typically include genetic sequences encoding at least the P2X7 receptor binding domain operably joined to transcriptional control elements which are exogenous to the P2X7 sequences (and possibly exogenous to the genome of the target cell). These transcriptional control elements will include a transcriptional promoter region and optionally enhancer sequences. Examples of transcriptional promoters and enhancers that are incorporated into a construct include, but are not limited to, cell- or tissue-specific promoters, inducible promoters, and regulatable promoters. Specific examples include, but are not limited to, the herpes simplex thymidine kinase promoter, the cytomegalovirus (CMV) promoter/enhancer, SV40 promoters, PGK promoter, metallothionein promoter, adenovirus late promoter, vaccinia virus 7.5K promoter, avian beta globin promoter, histone promoters (*e.g.,* mouse histone H3-614 promoter), beta actin promoter, and the cauliflower mosaic virus 35S promoter (see generally, Sambrook et al., *Molecular Cloning,* Vols. I-III, Cold Spring Harbor Laboratory Press, Cold Spring Harbor, New York (1989), and *Current Protocols in Molecular Biology,* John Wiley & Sons (1989-2000 editions)).

### 9. Methods of Treatment for Skeletal Disorders

In another aspect, the invention provides methods for increasing bone mass and/or bone mineral density in a mammal in need of such treatment by administering an amount of an agonist of a P2X7 receptor effective to increase bone mass and/or bone mineral density in the mammal. In another aspect, the invention provides methods for treating a mammal having a skeletal disorder characterized by decreased bone mass and/or bone mineral density by administering an amount of an agonist of a P2X7 receptor effective to increase bone mass and/or bone mineral density in the mammal. In some embodiments of these methods, the mammal is afflicted with, or at substantial risk of, a disorder such as osteoporosis, periodontitis, orthopedic osteolysis, inflammatory bone diseases, or a bone fracture or other bone injury. In embodiments in which the disorder or damage to bone is localized (*e.g.*, periodontitis or a bone fracture), it is preferred that administration of the P2X7 receptor agonist also be localized, as by administration of a paste, gel, or implanted extended-release formulation at or near the site of the disorder or damage.

The P2X7 receptor agonists are administered as therapeutic compositions in physiologically and/or pharmaceutically acceptable carriers. For example, the P2X7 receptor agonists can be administered to patients generally as described in *Harrison's Principles of Internal Medicine,* 14th Edition, McGraw-Hill, NY (1998). The characteristics of the carrier will depend on the route of administration.

Administration can be bolus, intermittent, or continuous, depending on the condition and response, as determined by those with skill in the art. In some preferred embodiments, the agonist is administered locally *(e.g.,* intralesionally) and/or systemically. The term "local administration" refers to delivery to a defined area or region of the body, while the term "systemic administration" is meant to encompass delivery to the whole organism by oral ingestion, or by intramuscular, intravenous, subcutaneous, or intraperitoneal injection.

Such a composition can contain, in addition to the P2X7 receptor agonist and carrier, diluents, fillers, salts, buffers, stabilizers, solubilizers, and other materials well known in the art. The pharmaceutical composition can also contain other active factors and/or agents which enhance bone formation or maintenance. Such additional factors and/or agents can be included in the pharmaceutical composition to produce a synergistic effect with the P2X7 receptor agonist, or to minimize side-effects caused by the agonist.

The pharmaceutical compositions can be in the form of a liposome in which the P2X7 receptor agonist is combined, in addition to other pharmaceutically acceptable carriers, with amphipathic agents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. One particularly useful lipid carrier is lipofectin. Preparation of such liposomal formulations is within the level of skill in the art, as disclosed, for example, in U.S. Patent No. 4,235,871; U.S. Patent No. 4,501,728; U.S. Patent No. 4,837,028; and U.S. Patent No. 4,737,323. The pharmaceutical composition can further include compounds such as cyclodextrins and the like which enhance delivery of agents into cells, or slow release polymers.

In practicing the methods of treatment of the present invention, a therapeutically effective amount of one, two, or more P2X7 receptor agonists is administered to a subject afflicted with a disease or disorder characterized by decreased bone mass and/or bone mineral density. The agonists can be administered in accordance with the method of the invention either alone or in combination with other known therapies for the disease or disorder. When co-administered with one or more other therapies, the P2X7 receptor agonist can be administered either simultaneously with the other treatment(s), or sequentially. If administered sequentially, the attending physician will decide on the appropriate sequence of administering the P2X7 receptor agonist in combination with the other therapy.

Administration of the P2X7 receptor agonist in a pharmaceutical composition to practice the methods of the invention can be carried out in a variety of conventional ways, such as implantation, oral ingestion, inhalation, or cutaneous, subcutaneous, intramuscular, or intravenous injection.

If P2X7 receptor agonists are administered locally or regionally (*e.g.*, to the site of a lesion) as opposed to systemically, normal tissue uptake should be reduced. P2X7 receptor agonists may be administered locally or regionally by admixing the agonist in a biocompatible matrix material and implanting or applying the mixture at or near a site to be treated. For example, biocompatible polymers such as polylactic acid (PLA), polyglycolic acid (PGA), poly(co-lactic- glycolic) acid (PLGA), poly(ethylene glycol) (PEG), and the like can be formed into bioerodable implants which contain a P2X7 receptor agonist. Alternatively, various poultices or waxes known in the art can be admixed with a P2X7 receptor agonist and applied to a site within the body *(e.g.,* a periodontal lesion). In addition, methods of encapsulating P2X7 receptor agonists in liposomes and targeting these liposomes to selected tissues by inserting proteins into the liposome surface can be utilized (Pagnan et al. (2000), *J. Natl. Can. Inst.* 92:253-61; Yu et al. (1999), *Pharm. Res.* 16:1309-15).

When a therapeutically effective amount of a P2X7 receptor agonist is administered orally, the P2X7 receptor agonist will be in the form of a tablet, capsule, powder, solution or elixir. When administered in tablet form, the pharmaceutical composition can additionally contain a solid carrier such as a gelatin or an adjuvant. The tablet, capsule, and powder can contain from about 5 to 95% P2X7 receptor agonist and preferably from about 25 to 90% P2X7 receptor agonist. When administered in liquid form, a liquid carrier such as water, petroleum, oils of animal or plant origin such as peanut oil, mineral oil, soybean oil, sesame oil, or synthetic oils can be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of the P2X7 receptor agonist and preferably from about 1 to 50% P2X7 receptor agonists.

When a therapeutically effective amount of a P2X7 receptor agonist is administered by intravenous, subcutaneous, intramuscular, or intraperitoneal injection, the P2X7 receptor agonist will be in the form of a pyrogen-free, parenterally acceptable aqueous solution. The preparation of such parenterally acceptable solutions, having due regard to pH, isotonicity, stability, and the like, is within the skill in the art. A preferred pharmaceutical composition for intravenous, subcutaneous, intramuscular, or intraperitoneal injection should contain, in addition to the P2X7 receptor agonist, an isotonic vehicle. The pharmaceutical composition can also contain stabilizers, preservatives, buffers, antioxidants, or other additives known to those of skill in the art.

The amount of P2X7 receptor agonist in the pharmaceutical composition will depend upon the nature and severity of the condition being treated, and on the nature of prior treatments which the patent has undergone. Ultimately, the attending physician will decide the amount of P2X7 receptor agonist with which to treat each individual patient. Similarly, the duration of intravenous therapy using the pharmaceutical composition will vary depending on the severity of the disease being treated and the condition and potential idiosyncratic response of each individual patient. Ultimately the attending physician will decide on the appropriate duration of intravenous therapy using the pharmaceutical composition of the present invention. Initially, the attending physician can administer low doses of the P2X7 receptor agonist and observe the patient's response. Larger doses of P2X7 receptor agonists can be administered until the optimal therapeutic effect is obtained for the patient, and at that point the dosage is not increased further.

It is contemplated that the various pharmaceutical compositions used to practice the methods of the present invention should contain about 10 µg to about 20 mg of P2X7 receptor agonists per kg body or organ weight.

In order that the invention described herein may be more fully understood, the following examples are set forth. It should be understood that these examples are for illustrative purposes only and are not to be construed as limiting the present invention in any manner.

### EXAMPLE 1

### Bone Morphology in P2X7 Receptor-Deficient Mice

To directly address the role of the P2X7 receptor in regulating bone mass, the bone phenotype of a P2X7 receptor-deficient mouse line was characterized using peripheral quantitative computerized tomography (pQCT) and standard bone histomorphometric methods. The differences in bone mass and bone structures were compared with wild-type controls. These differences demonstrate that the P2X7 receptor plays a role in increasing bone bass and in regulating cancellous bone architecture.

### A. Study Protocol

A P2X7 receptor knock-out (KO) (hereafter P2X7RKO) mouse was generated as previously described (Solle et al. (2001), *J. Biological Chemistry,* 276:125-132). Thirteen female wild-type (WT) mice and thirteen P2X7RKO mice were weighed and necropsied at 8 weeks of age. The right femur from each mouse was analyzed by pQCT, and the volumetric total, trabecular and cortical bone mineral content and density were determined. The left femur from each mouse was processed to 4 µm sections for bone histomorphometric analysis.

### B. Peripheral quantitative computerized tomography (pQCT)

The right femur from each mouse was analyzed at the distal femoral metaphysis, a primary cancellous bone site, and the femoral diaphysis, a cortical bone site. Excised femurs were scanned with a pQCT X-ray machine (Stratec XCT Research M, Norland Medical Systems, Fort Atkinson, WI) with software version 5.40. A 1 mm thick cross section of the distal femur metaphysis was taken 2.5 mm proximal from the distal end, and a 1 mm thick cross section of the mid-femoral diaphysis was taken 7 mm proximal from the distal end with a voxel size of 0.10 mm. Cortical bone was defined and analyzed using contour mode 2 and cortical mode 4. An outer threshold setting of 340 mg/cm³ was used to distinguish the cortical shell from soft tissue, and an inner threshold of 529 mg/cm³ to distinguish cortical bone along the endocortical surface. Trabecular bone was determined using peel mode 4 with a threshold setting of 655 mg/cm³ to distinguish cortical and subcortical from cancellous bone. An additional concentric peel of 1% of the defined cancellous bone was used to ensure cortical and subcortical bone was eliminated from the analysis. Volumetric content, density, and area were determined for total, trabecular, and cortical bones as previously described (Beamer et al. (1996), *Bone;* 18:397-403). In addition, cortical periosteal and endosteal circumference was determined. Using the above methods, it was determined that the *ex vivo* precision of volumetric content, density and area of total bone, trabecular bone, and cortical regions ranged from 0.99% to 3.49% with repositioning.

### C. Cancellous bone histomorphometry of distal femoral metaphysis

Undecalcified, methyl methacrylate embedded longitudinal sections of distal femoral metaphysis of 4 µm thickness were prepared for histomorphometry as described previously (Jee et al. (1997), in *Handbook of Bone Morphology,* Takahashi, ed., Niigata City, Japan: Nishimusa, pp 87-112). The sections were stained with modified Masson's Trichrome stain. An Image Analysis System (Osteomeasure, Inc., Atlanta, GA) was used for histomorphometric analysis. Histomorphometric measurements were performed in cancellous bone tissue of the distal femoral metaphyses between 0.5 mm and 2.5 mm proximal to the growth plate-epiphyseal junction, and extended to the endocortical surface in the lateral dimension. Measurements and calculations related to trabecular bone volume and structure included trabecular bone volume, trabecular thickness, trabecular number, and trabecular separation as described previously (Jee et al. (1997), *supra*).

### D. Results

There was no significant difference in body weight in P2X7RKO (19.33+/- 0.4 grams) compared with WT controls (19.85+/- 0.7 grams). Similarly, there was no significant difference in femoral length between P2X7RKO (14.83+/- 0.37 mm) and WT controls (14.55+/- 0.47 mm). However, compared with WT controls, P2X7RKO mice had significantly lower volumetric cortical-subcortical bone mineral content (-13%), volumetric cortical-subcortical bone mineral density (-7%), volumetric cortical bone mineral content (-13%), and volumetric cortical bone mineral density (-5%) at the distal femoral metaphysis. Similarly, P2X7RKO mice had significantly lower volumetric total bone mineral content (-12%), volumetric total bone mineral density (-11%), volumetric cortical-subcortical bone mineral content (-10%), volumetric trabecular bone mineral content (-26%), volumetric cortical bone mineral area (-8%), and volumetric cortical bone mineral content (-10%), periosteal circumference (-6%), and endocortical circumference (-9%) at the femoral shafts when compared with WT controls.

Distal femoral metaphyseal cancellous bone histomorphometric analysis showed that P2X7RKO mice had significantly lower trabecular bone volume (-32%), trabecular bone thickness (-18%), and trabecular bone number (-19%), and significantly higher trabecular separation (+40%).

These data demonstrate that although body weight and bone length do not differ between mice lacking the P2X7 receptor and wild-type controls, lower cancellous and cortical bone mass and poor cancellous bone architecture are found in mice lacking the P2X7 receptor.

### EXAMPLE 2

### Role of P2X7 Receptor on Regulation of Skeletal Growth and Metabolism in vivo.

Skeletal growth, development, metabolism, and maintenance are controlled by bone growth, modeling and remodeling. Osteoclasts and osteoblast play important roles in regulating bone growth, modeling and remodeling. The P2X7 receptor (P2X7R) is an ATP-gated ion channel expressed by monocytes, macrophages, osteoclasts and subpopulation of osteoblasts. To directly address the role of this receptor in regulating bone mass, we characterized the bone phenotype of P2X7R-deficient mouse line using peripheral quantitative computerized tomography (pQCT) and standard bone histomorphometric methods. The differences in bone mass and bone structures were compared with wild-type (WT) controls.

### A. Study Protocol

Male and female wild-type (WT) mice and P2X7RKO mice were weighed and necropsied at 2, 5, 9 and 15 months of age as seen in Table 1.

**Table 1.**

| Study Protocol | | | |
|---|---|---|---|
| Phenotypes | Sex | Age (mo.) | Number of animals |
| WT | Male | 2 | 15 |
| P2X7R KO | Male | 2 | 19 |
| | | | |
| WT | Male | 5 | 15 |
| P2X7R KO | Male | 5 | 16 |
| | | | |
| WT | Male | 9 | 14 |
| P2X7R KO | Male | 9 | 13 |
| | | | |
| WT | Male | 15 | 21 |
| P2X7R KO | Male | 15 | 15 |
| | | | |
| WT | Female | 2 | 13 |
| P2X7R KO | Female | 2 | 13 |
| | | | |
| WT | Female | 5 | 6 |
| P2X7R KO | Female | 5 | 9 |
| | | | |
| WT | Female | 9 | 14 |
| P2X7R KO | Female | 9 | 13 |
| | | | |
| WT | Female | 15 | 23 |
| P2X7R KO | Female | 15 | 23 |

All mice were s.c. injection with 0.5 mg of calcein for fluorescent bone label 2 and 12 days before necropsy. The right femoral length was determined. Then, the distal femoral metaphysis and tibial shafts from each mouse was analyzed by pQCT, and volumetric total, trabecular and cortical bone mineral content and density were determined. The left femur from each mouse was processed to 4µm sections for distal femoral metaphyseal cancellous bone histomorphometric analysis. The left proximal tibial metaphyseal cancellous bone and tibial shaft cortical bone histomorphometric analysis were performed.

### B. Peripheral quantitative computerized tomography (pQCT)

The right femur from each mouse was analyzed in two bone sites, the distal femoral metaphysis, a primary cancellous bone site, and the femoral diaphysis, a cortical bone site. Excised femurs were scanned with a pQCT X-ray machine (Stratec XCT Research M, Norland Medical Systems, Fort Atkinson, WI.) with software version 5.40. A 1 mm thick cross section of the distal femur metaphysis was taken at 2.5 mm proximal from the distal end, and a 1 mm thick cross section of the mid-femoral diaphysis was taken at 7 mm proximal from the distal end with a voxel size of 0.10 mm. Cortical bone was defined and analyzed using contour mode 2 and cortical mode 4. An outer threshold setting of 340 mg/cm³ was used to distinguish the cortical shell from soft tissue and an inner threshold of 529 mg/cm³ to distinguish cortical bone along the endocortical surface. Trabecular bone was determined using peel mode 4 with a threshold setting of 655 mg/cm³ to distinguish cortical and subcortical from cancellous bone. An additional concentric peel of 1% of the defined cancellous bone was used to ensure cortical and subcortical bone is eliminated from the analysis. Volumetric content, density, and area were determined for total, trabecular, and cortical bones (Beamer WG, Donahue LR, Rosen CJ, Baylink DJ Genetic variability in adult bone density among inbred strains of mice. Bone 1996;18:397-403). In addition, cortical periosteal and endosteal circumference was also determined. Using the above setting, we have determined that the *ex vivo* precision of volumetric content, density and area of total bone, trabecular, and cortical regions ranged from 0.99% to 3.49% with repositioning.

### C. Cancellous bone histomorphometry of distal femoral metaphysis

Undecalcified, methyl methacrylate embedded longitudinal sections of distal femoral metaphysis at 4 µm thickness were prepared for histomorphometry as described previously (Jee WSS, Li XJ, Inoue J, Jee KW, Haba T, Ke HZ, Setterberg RB, Ma YF Histomorphometric assay of the growing long bone. In: Takahashi H., ed. Handbook of Bone Morphology. Niigata City, Japan: Nishimusa, 1997, pp 87-112). The sections were stained with modified Masson's Trichrome stain. An Image Analysis System (Osteomeasure, Inc., Atlanta, GA) was used for histomorphometric analysis. Histomorphometric measurements were performed in cancellous bone tissue of the distal femoral metaphyses between 0.5 mm and 2.5 mm proximal to the growth plate-epiphyseal junction, and extended to the endocortical surface in the lateral dimension. Measurements and calculations related to trabecular bone volume and structure included trabecular bone volume (TBV), thickness (Tb.Th), number (Tb.N), and separation (Tb.Sp) as described previously (Jee WSS, Li XJ, Inoue J, Jee KW, Haba T, Ke HZ, Setterberg RB, Ma YF Histomorphometric assay of the growing long bone. In: Takahashi H., ed. Handbook of Bone Morphology. Niigata City, Japan: Nishimusa, 1997, pp 87-112).

### D. Cancellous bone histomorphometry of proximal tibial metaphysis

Undecalcified, methyl methacrylate embedded longitudinal sections of proximal tibial metaphysis at 4 µm thickness were prepared for histomorphometry as described above. Both static and dynamic histomorphometric parameters were determined.

### E. Cortical bone histomorphometry of tibial shafts

Undecalcified, methyl methacrylate embedded longitudinal sections of tibial shaft at 20 µm thickness were prepared for histomorphometry as described previously (Jee WSS, Li XJ, Inoue J, Jee KW, Haba T, Ke HZ, Setterberg RB, Ma YF Histomorphometric assay of the growing long bone. In: Takahashi H., ed. Handbook of Bone Morphology. Niigata City, Japan: Nishimusa, 1997, pp 87-112). Both static and dynamic histomorphometric parameters were determined.

### F. Results

Both male and female P2X7RKO exhibited low bone mass phenotype when compared with their age-match, wild-type littermates. The experimental findings with the male mice are presented and described here in detail.

**Femoral length:** In male mice, femoral length did not differ significantly between P2X7RKO and WT controls in all ages (Figures 1 and 2), with the exception at 5 months of age that shows P2X7RKO had significantly shorter femur than WT controls, indicating that P2X7R plays a very minimal role in longitudinal bone growth. However, x-ray images showed that P2X7RKO mice had significantly smaller femoral diameters than the WT controls (Figure 2)

**Distal femoral pQCT analysis:** In male mice, pQCT analysis of distal femoral metaphysis show that P2X7RKO mice had significantly lower total bone area, total bone mineral content, trabecular content, cortical content at both 2 and 5 months of age, as compared with WT controls. At 9 month, total area, total bone mineral content and cortical content remained significantly lower in P2X7RKO as compared with WT controls, while trabecular content did not show any difference between P2X7RKO and WT due to the age-related decrease in trabecular content in the WT mice (Figure 2). At 15 months of age, there was no significant difference in total bone mineral content, trabecular content and cortical content between P2X7RKO and WT due to the age-related decline in WT mice. Total area was still maintained at the level that was significant lower in P2X7RKO than in WT (Figures 3 and 4).

**Femoral shaft pQCT analysis:** In male mice, pQCT analysis of femoral shafts show that total area, total bone mineral content, cortical bone area and cortical content were significantly lower in P2X7RKO compared with age-match WT controls at 2, 5, 9, and 15 months of age (Figures 3 and 5).

**Cortical bone histomorphometric analysis of tibial shafts:** At 2 months of age, P2X7RKO male mice had significant lower total tissue area (-23%), cortical bone area (-23%) and marrow cavity area (-25%) as compared with the WT controls. The cortical bone mass and structure changes are accompanied with a significant lower periosteal mineralizing surface (-28%) in the P2X7RKO compared with the WT. The above differences maintained up to 15 months of age between P2X7RKO and WT controls. The data indicate that P2X7RKO male mice had lower cortical bone mass due to lower periosteal bone formation compared with the WT controls.

**Cancellous bone histomorphometric analysis of distal femoral metaphysis:** Distal femoral metaphyseal cancellous bone histomorphometric analysis shown that P2X7RKO male mice had significantly lower osteoblast surface (-51%) and significant higher osteoclast surface (+64%) at 9 months of age. The data indicate that P2X7RKO male mice had higher bone resorption and lower bone formation than WT controls.

**Cancellous bone histomorphometric analysis of proximal tibial metaphysis:** Proximal tibial metaphyseal cancellous bone histomorphometric analysis show that P2X7RKO male mice had significantly lower trabecular bone volume (-40%) and significant higher osteoclast surface (+54%) and osteoclast number (+152%) at 9 months of age. The data indicate that P2X7RKO male mice had higher bone resorption than WT controls.

These data show that although bone length do not differ between mice lacking P2X7 receptor and wild-type controls, lower cancellous and cortical bone mass and poor cancellous bone architecture are found in mice lacking P2X7 receptor. Further, mice lacking P2X7 receptor had higher cancellous bone resorption and lower cancellous and cortical bone formation. The data indicate that P2X7 receptor plays an important role in bone mass augmentation and maintenance.

### EXAMPLE 3

### Peritoneal Macrophage Isolation for Cell-Based Assays

Peritoneal macrophages are harvested by injecting 5 ml of Roswell Park Memorial Institute (RPMI) medium containing 5% fetal bovine serum (FBS) into the peritoneal cavity of an animal *(e.g.,* mouse), immediately after the animals are sacrificed by cervical dislocation. The injected medium is dispersed throughout the peritoneal cavity by rubbing the external surface, a hole in the skin covering the peritoneum is made to gain access to the cavity, and the injected fluid is recovered with the aid of a transfer pipette. Lavage fluids from multiple animals are pooled and the cells are collected by centrifugation (300 x g). The cell pellets are washed twice by centrifugation in RPMI containing 5% FBS.

### EXAMPLE 4

### P2X7 Receptor-Mediated Macromolecule Uptake

One activity of the P2X7 receptor is to facilitate translocation of large organic molecules such as the fluorescent dye YoPro Yellow in response to ATP activation (Virginio at al. (1999) *J. Physiol*. 519:335-346). When peritoneal macrophages isolated from wild-type animals are activated with 5 mM ATP in the presence of extracellular YoPro Yellow, a time dependent increase in fluorescence intensity is observed. This increase in fluorescence results from internalization of the dye molecules followed by their binding to DNA; when bound to DNA, the fluorescence intensity increases. In the absence of ATP, no significant increase in fluorescence intensity is observed, indicating that the plasma membrane is impermeable to YoPro Yellow in the absence of the nucleotide triphosphate. According to one embodiment of the present invention, compounds are tested for their ability modulate P2X7 receptor-mediated translocation of large organic molecules such as fluorescent dyes, with or without concomitant exposure to ATP.

When assaying P2X7 receptor function using isolated macrophages, the peritoneal macrophages are washed with isotonic medium (15 mM HEPES, pH 7.2, 135 mM NaCI, 5 mM KCI, 18 mM CaCl₂, 08 mM MgCl₂) by centrifugation and the resulting cell pellet is suspended in isotonic medium to achieve a final cell concentration of about 1x10⁶ cells/ml. Fifty µl of this cell suspension is placed into the wells of a plate (*e.g.*, Microfluor "B" U-bottom plate, Dynatech, Chantilly, VA), and 50 µl of a fluorescent dye (*e.g.,* 2 µM YoPro Yellow, Molecular Probes, Eugene, OR) dissolved in the isotonic medium is added to each well. Test compounds or control vehicles are added to the wells with or without the addition of 5 mM ATP. Fluorescence is monitored as a function of time at 37° C. For YoPro Yellow dye, excitation is at 450 nm and emission is measured at 530 nm. Test compounds which stimulate P2X7 receptor activity increase fluorescence as compared to control samples that lack test compound.

### EXAMPLE 5

### IL-1β Post-translational Processing In Isolated Macrophages

Peritoneal macrophages isolated from animals such as mice are stimulated with lipopolysaccharide (LPS) and labeled with [³⁵S]methionine. These radiolabeled cells are then cultured in the absence or presence of a secretory stimulus such as 5 mM ATP, after which cells and media are harvested separately, cells are solubilized by detergent extraction, and IL-1β is recovered from the media and cell extracts by immunoprecipitation.

The macrophages are seeded on plates (*e.g.*, 1 x 10⁶ cells per well) and stimulated with 1 µg/ml *E. coli* LPS (Serotype 055 B5 obtained from Sigma; St. Louis, MO) for 75-90 minutes. The cells are then rinsed with 2 ml of methionine-free RPMI medium containing 100 units/ml penicillin, 100 µg/ml streptomycin, 1% dialyzed FBS, 1 µg/ml LPS, and 25 mM HEPES at pH 7.3 ("pulse medium"). One ml of pulse medium containing 83 µCi/ml of ³⁵S-methionine (Amersham Corp., Chicago, IL) is then added to each well, and the cells are labeled at 37°C for 1 hour. The labeled cells are subsequently rinsed twice with RPMI 1640 medium containing 100 units/ml penicillin, 100 µg/ml streptomycin, 1% FBS, 2 mM glutamine, 1 µg/ml LPS and 25 mM HEPES at pH 7.3 ("chase medium"). One ml of chase medium with or without a test compound and/or 5 mM ATP is added to each well, and the cells are chased at 37°C for 30 minutes. Media are harvested and clarified by centrifugation (6000 x g for 5 minutes) to remove cells and/or cell debris. Cell monolayers are suspended in 1 ml of a lysis buffer composed of 1 % Triton X-100, 150 mM NaCI, 25 mM HEPES, pH 7, 0.1 mM PMSF, 1 mg/ml ovalbumin, 1 mM iodoacetic acid, 1 µg/ml pepstatin, and 1 µg/ml leupeptin. Clarified media samples are adjusted to the same final Triton X-100 and protease inhibitor concentrations by addition of aliquots from concentrated stocks of these reagents. After a 30 minute incubation on ice, all samples are clarified by centrifugation at 45,000 rpm for 30 minutes in a TLA-45 rotor (Beckman, Palo Alto, CA), the resulting supernatants are recovered, and IL-1β is immunoprecipitated from the samples using a goat anti-murine IL-1β serum. Immunoprecipitates are fractionated by SDS gel electrophoresis, and the quantity of radioactivity associated with individual IL-1β polypeptide species is determined by scanning dried gels with a Phosphoimager.

### EXAMPLE 6

### IL-1β Post-translational Processing In Vivo

Peritoneal macrophages of animals such as mice exposed to bacterial lipopolysaccharide (LPS) *in viv*o require a secretion stimulus such as 5 mM ATP to elicit efficient externalization of mature IL-1β (Griffiths et al. (1995) *J. Immunol.* 154:2821-2828). To determine the degree of IL-1β processing *in vivo*, animals are primed with LPS, and 2 hours later receive an ip injection of PBS with or without ATP, and/or with or without a test compound. Peritoneal lavage fluids from these animals are then assessed for IL-1β content by an enzyme-linked immuno-sorbent assay (ELISA).

Groups of mice are injected intraperitoneally (ip) with 1 µg of LPS. Two hours after LPS injection, the mice are injected ip with phosphate buffered saline (PBS) pH 7 with or without 30 mM ATP (adjusted to pH 7) with or without the test compound. Mice are sacrificed 30 minutes or 120 minutes after the ATP or PBS injection and peritoneal cavities are lavaged with 3 ml of RPMI with 5% FBS. Samples of peritoneal lavages are spun down, and the supernatants are collected and tested for the presence of IL-1β and IL-6 using ELISAs (Amersham Life Sciences, Arlington Heights, VA and Endogen, Woburn, MA, respectively).

### EXAMPLE 7

### Characterization of In Vivo Cytokine Production Capabilities

IL-1 signaling may lead to the production of other cytokines such as IL-6 (Allen et al. (2000), *J. Exp. Med.* 191:859-869). To assess this cascade effect, animals, such as mice, are primed by an injection of LPS (or PBS as a control), and two hours later an injection of ATP or a test compound (or PBS as a control) is administered to promote IL-1 post-translational processing. The animals are incubated for an additional period (*e.g*., 4 hours) and peritoneal lavages are collected several times during the course of the incubation and analyzed for cytokines by ELISA. Mice that receive an initial priming injection of LPS followed by PBS yield levels of IL-6 at 1 and 2 hours (4 to 5 ng/ml) that are elevated above those recovered from mice injected with a combination of PBS and ATP. By 4 hours, however, IL-6 levels recovered from the LPS-PBS treated animals returned to baseline. On the other hand, LPS-primed mice that subsequently are challenged with ATP demonstrate a dramatic increase in lavage fluid IL-6, reaching a peak value >25 ng/ml at the 4 hour time point.

### EXAMPLE 8

### Partial Receptor Purification

Murine peritoneal macrophage cell pellets are washed once in a cavitation buffer (25 mM HEPES, pH 7, 30 mM NaCl, 1 mM EDTA, 1 mM dithiothreitol, 1 µg/ml leupeptin, and 1 µg/ml pepstatin) by centrifugation. Cell pellets then are suspended in 2.5 ml of cavitation buffer and the cells are disrupted by nitrogen cavitation (15 minutes on ice at 750 psi). The resulting cell lysates are adjusted to 0.1% saponin, incubated on ice for 30 minutes, and cell membranes subsequently are recovered by centrifugation (50,000 rpm for 30 minutes at 4°C in a Beckman Ti70 rotor). The membrane pellet is suspended in 2 ml cavitation buffer with the aid of a glass tube-teflon pestle homogenizer. The membranes again are collected by centrifugation after which the pellets are suspended in 100 µl of 2 X Laemmli sample buffer. The protein is further purified by separation on a 4-20% Tris-Glycine gel (Novex, San Diego, CA), and/or immunoprecipitation using anti-P2X7 receptor serum (Alomone, Jerusalem, Israel).

### EXAMPLE 9

### Assays for Osteoclast Differentiation and Apoptosis

A bone marrow osteoclast differentiation culture system (e.g., mouse or rat) is established as previously described (Takahashi et al. (1988), *Endocrinology* 122:1373-1381; Grasser et al. (1997), *J. Cell. Biochem.* 65:159-171; Ke et al. (1998), *Endocrinology* 139:2068-2076). In brief, bone marrow is collected, the cells are plated at a density of 5 x 10⁵/cm², and then the cells are treated with either vehicle or a test compound with or without a P2X7 receptor ligand. The cell cultures are maintained for 6 days with 50% of the media containing fresh compounds being replaced on the third day, followed by osteoclast staining and quantitative observation on the sixth day. To visualize osteoclasts formed in culture, histochemical staining for tartrate-resistant acid phosphatase (TRAP) is performed. To identify apoptotic cells, fragmented nuclear DNA is labeled by TDT-mediated dUTP nick-end labeling (TUNEL) according to manufacturer's directions (Boehringer-Mannheim, Indianapolis, IN). On day 3 of culture, p53 and CD61 co-localization is done as previously described using p53- and CD61-specific monoclonal antibodies (Oncogene Sciences, Cambridge, MA, and Pharmingen, San Diego, CA, respectively). Labeled cells are viewed and counted under a microscope (*e.g.*, Olympus BH-2).

### Equivalents

The present invention is not to be limited in scope by the particular embodiments described herein. Rather, various modifications of the invention in addition to those described herein will become apparent to those skilled in the art from the foregoing description and accompanying figures. Such modifications are intended to fall within the scope of the appended claims.

## Claims

1. A method for screening compounds to identify therapeutic candidates for increasing bone mass and/or bone mineral density, said method comprising:
contacting a cell that expresses a P2X7 receptor with a test compound under conditions which, but for the presence of the compound, permit binding of said P2X7 receptor to a P2X7 receptor ligand; and
detecting an indicator of P2X7 receptor activity;
wherein an increase in said indicator indicates that said compound is a candidate for increasing bone mass and/or bone mineral density by interacting with a P2X7 receptor *in vivo.*

2. A method for screening compounds to identify therapeutic candidates for modulating bone mass and/or bone mineral density, said method comprising:
contacting a cell that expresses a P2X7 receptor with a test compound and a P2X7 receptor ligand under conditions which, but for the presence of the compound, permit binding of said P2X7 receptor to said P2X7 receptor ligand; and
detecting an increase or decrease in an indicator of P2X7 receptor activity;
wherein an increase or decrease in said indicator indicates that said compound is a candidate for modulating bone mass and/or bone mineral density by interacting with a P2X7 receptor *in vivo*.

3. The method of claim 1 or 2, wherein said indicator is selected from the group consisting of increased cell permeability; increased intracellular accumulation of a macromolecule; increased IL-1α, IL-1β, or IL-18 post-translational processing in activated inflammatory cells; increased IL-6 production in inflammation-induced cells; increased L-selectin shedding; stress kinase activation; and lymphoproliferation.

4. The method of claim 1 or 2, wherein said cell is an osteoclast progenitor and said indicator is differentiation into an osteoclast.

5. The method of claim 1 or 2, wherein said cell is an osteoclast and said indicator is increased resorption pit formation or increased apoptosis.

6. A method for screening compounds to identify therapeutic candidates for modulating bone mass and/or bone mineral density, said method comprising:
contacting a compound with a cell expressing a P2X7 receptor binding domain on the surface of said cell under conditions which, but for the presence of the compound, permit binding of said P2X7 receptor binding domain to a P2X7 receptor ligand; and
detecting binding between said P2X7 receptor binding domain and said compound;
wherein binding between said P2X7 receptor binding domain and said compound indicates that said compound is a candidate for modulating bone mass and/or bone mineral density by interacting with a P2X7 receptor *in vivo.*

7. A method for screening compounds to identify therapeutic candidates for modulating bone mass and/or bone mineral density, said method comprising:
contacting a compound with a cell expressing a P2X7 receptor binding domain on the surface of said cell and a P2X7 receptor ligand under conditions which, but for the presence of the compound, permit binding of said P2X7 receptor binding domain to said P2X7 receptor ligand; and
detecting binding between said P2X7 receptor binding domain and at least one of said compound and said P2X7 receptor ligand;
wherein the detection of binding between said P2X7 receptor binding domain and said compound, or the detection of increased or decreased binding between said P2X7 receptor binding domain and said P2X7 receptor ligand, indicates that said compound is a candidate for modulating bone mass and/or bone mineral density by interacting with a P2X7 receptor *in vivo*.

8. A method for screening compounds to identify therapeutic candidates for modulating bone mass and/or bone mineral density, said method comprising:
contacting a test compound with a cell expressing a coding sequence under the control of a P2X7 receptor gene transcriptional control element; and
detecting whether said compound increases or decreases expression of said coding sequence;
wherein an increase or decrease in expression of said coding sequence indicates that said compound is a candidate for modulating bone mass and/or bone mineral density by modulating P2X7 receptor expression *in vivo.*

9. The method of claim 8, wherein said cell is transformed with a reporter gene coding sequence operably joined to a P2X7 receptor gene transcriptional control element, wherein an increase or decrease in reporter gene expression indicates that said compound is a candidate for modulating bone mass and/or bone mineral density by modulating P2X7 receptor expression *in vivo*.

10. A method for screening compounds to identify therapeutic candidates for modulating bone mass and/or bone mineral density, said method comprising:
providing a compound;
administering said compound to a non-human animal that expresses a P2X7 receptor; and
detecting whether said compound increases or decreases an indicator of P2X7 receptor activity in said animal;
wherein an increase in said indicator indicates that said compound is a candidate for increasing bone mass and/or bone mineral density by increasing P2X7 receptor activity *in vivo,* and wherein a decrease in said indicator indicates that said compound is a candidate for decreasing bone mass and/or bone mineral density by decreasing P2X7 receptor activity *in vivo*.

11. The method of claim 10, wherein said animal is a transgenic non-human animal which has been transformed with an exogenous nucleic sequence encoding a P2X7 receptor which is expressed in said animal, or a descendant of such an animal.

12. The method of claim 10, wherein said indicator is selected from the group consisting of increased cell permeability; increased intracellular accumulation of a macromolecule; increased IL-1α, IL-1β, or IL-18 post-translational processing in activated inflammatory cells; increased IL-6 production in inflammation-induced cells; increased L-selectin shedding; stress kinase activation; and lymphoproliferation.

13. The method of claim 10, wherein said indicator is differentiation of osteoclast progenitors into osteoclasts.

14. The method of claim 10, wherein said indicator is increased resorption pit formation or increased apoptosis in osteoclasts.

15. The method of claim 10, wherein said indicator is a phenotypic change selected from the group consisting of bone mineral density, bone mineral content, trabecular bone number, trabecular bone separation, bone mineral area, bone volume, bone thickness, and bone circumference.

16. A method for screening compounds to identify therapeutic candidates for modulating bone mass and/or bone mineral density, said method comprising:
contacting a compound with a binding domain of a P2X7 receptor under conditions which, but for the presence of the compound, permit binding of said P2X7 receptor binding domain to a P2X7 receptor ligand; and
detecting binding between said P2X7 receptor binding domain and said compound;
wherein binding between said P2X7 receptor binding domain and said compound indicates that said compound is a candidate for modulating bone mass and/or bone mineral density by interacting with a P2X7 receptor *in vivo*.

17. A method for screening compounds to identify therapeutic candidates for modulating bone mass and/or bone mineral density, said method comprising:
contacting a compound with a binding domain of a P2X7 receptor and a P2X7 receptor ligand under conditions which, but for the presence of the compound, permit binding of said P2X7 receptor binding domain to said P2X7 receptor ligand; and
detecting binding between said P2X7 receptor binding domain and at least one of said compound and said P2X7 receptor ligand;
wherein the detection of binding between said P2X7 receptor binding domain and said compound, or the detection of decreased binding between said P2X7 receptor binding domain and said P2X7 receptor ligand, indicates that said compound is a candidate for modulating bone mass and/or bone mineral density by interacting with a P2X7 receptor *in vivo*.

18. Use of an agonist of a P2X7 receptor in the manufacture of a medicament to increase bone mass and/or bone mineral density in a mammal.

19. The use of claim 18, wherein said mammal is afflicted with, or at substantial risk of, a disorder selected from the group consisting of osteoporosis, periodontitis, orthopedic osteolysis, inflammatory bone diseases, and bone fracture.
